# EUROPEAN PATENT SPECIFICATION

(11) **EP 1 826 218 B1**
(45) Date of publication and mention of the grant of the patent: **01.01.2014**
(21) Application number: 05776879.8
(22) Date of filing: 30.08.2005
(51) Int. Cl.: C07K 16/24, C12N 15/02, C12P 21/08, A61K 39/395, A61P 37/00, A61P 3/10, A61P 29/00, A61P 19/02, G01N 33/53, G01N 33/15, C07K 14/705, G01N 33/564, G01N 33/74

(54) **ANTIHUMAN BAFF ANTIBODY**
ANTIHUMANE BAFF-ANTIKÖRPER
ANTICORPS ANTI-BAFF HUMAIN

(30) Priority: 31.08.2004 US 605516 P
(43) Date of publication of application: 29.08.2007
(73) Proprietor: Kowa Company, Ltd., Nagoya-shi, Aichi-ken 460-8625 (JP); Takeuchi, Tsutomu, Tokyo 156-0044 (JP); Yoshimoto, Keiko, Tokyo 165-0034 (JP)
(72) Inventor: TAKEUCHI, Tsutomu, Tokyo, 1560044 (JP); YOSHIMOTO, Keiko, Tokyo 165-0034 (JP)
(74) Representative: Hiebl, Inge Elisabeth
(86) International application number: PCT/JP2005/015696
(87) International publication number: WO 2006/025345

(56) References cited:
- WO-A-03/016468
- WO-A-03/055979
- WO-A2-03/016468
- WO-A2-03/055979
- JP-A- 2002 535 285
- JP-A- 2004 509 615
- US-A1- 2002 150 579
- ZHANG J ET AL: "Cutting edge: a role for B lymphocyte stimulator in systemic lupus erythematosus" JOURNAL OF IMMUNOLOGY, AMERICAN ASSOCIATION OF IMMUNOLOGISTS, US, vol. 166, 1 January 2001 (2001-01-01), pages 6-10, XP002960695 ISSN: 0022-1767
- MOORE P A ET AL: "BLyS: member of the tumor necrosis factor family and B lymphocyte stimulator.", SCIENCE (NEW YORK, N.Y.) 9 JUL 1999 LNKD- PUBMED:10398604, vol. 285, no. 5425, 9 July 1999 (1999-07-09), pages 260-263, ISSN: 0036-8075

## Description

### Technical Field

The present invention relates to a novel antihuman BAFF antibody, preferably an antihuman BAFF monoclonal antibody for the use thereof in the prevention/treatment of an autoimmune disease. Moreover, the invention relates to the use of said antibody for diagnosis of an autoimmune disease. In addition, the present invention relates to a pharmaceutical composition for use in prophylaxis and therapy of an autoimmune disease such as systemic lupus erythematosus (SLE), chronic rheumatoid arthritis (RA), Sjogren's syndrome (SS), autoimmune diabetes, or an autoimmune disease accompanied by B-cell activation, which includes use of the antibody, preferably the monoclonal antibody, a diagnostic agent and in vitro diagnostic method comprising the same.
Further, the present invention relates to a method of quantifying BAFF and a method of screening a substance having an inhibiting action or an activating action on BAFF, which includes use of the antibody, preferably the monoclonal antibody.

### Background Art

It is known that BAFF (B cell activating factor belonging to the TNF family) is produced and secreted from T cells, monocytes/macrophages, dendritic cells and the like and regulates such as B-cell differentiation, activation, survival rate via three types of receptors on B cells (Moore et al. , Science, 285, 260-263 (1999)).

Human BAFF is a transmembrane form protein comprising 285 amino acids. There is a structural characteristic of trimer formation such as the presence of a cytoplasmic domain of 46 amino acids, an extracellular domain of 218 amino acids, and two N-glycosylation sites in its amino acid sequence. It is estimated that an extracellular domain of 152 amino acids from C- terminal is cleaved with a protease of Furin family and released in a soluble form. The amino acid sequence of human BAFF initially named Neutrokine α was disclosed as SEQ ID NO: 1 or 2in publicationofInternationalPatentApplication WO98/18921. Other names of human BAFF such as Kay, TNFSF13B, Blys, TALL-1, THANK and zTNF4 are also known.

BAFF-R, TACI (transmembrane activator and calcium modulator and cyclophilin ligand interactor), and BCMA (B cell maturation antigen) are known as BAFF receptors. BAFF-R and BCMA are expressed mainly in B cells, and TACI is expressed in B cells and activated T cells.

The physiological action of BAFF lies in regulation of B-cell differentiation, activation, survival rate and the like as described above, and is increasingly revealed in recent years to participate in pathologic condition. That is, it is reported that a mouse expressing BAFF in excess shows SLE-like symptoms such as increase in peripheral blood B cells, enlargement of lymph nodes and spleen, increase in IgG level in serum, antinuclear antibody production, deposition of immune complex in the kidney, albuminuria and nephritis (Mackay et al . , J. Exp. Med., 190, 1697-1710, (1999), and Khare et al. , Proc. Natl. Acad. Sci. USA 97, 3370-3375, (2000)). It was further reveled that this mouse also shows SS-like symptoms such as inflammation of salivary gland and destruction of salivary gland with advancing age (Groom et al., J. Clin. Invest., 109, 59-68, (2002)). An increase of BAFF level in serum in patients suffering from SLE, RA and SS is also reported (Groom et al., J. Clin. Invest., 109, 59-68, (2002); Zhang et al., J. Immunol., 166, 6-10, (2001); and Cheema et al., Arthritis Rheum., 44, 1313-1319, (2001)), and there are also many reports such as higher BAFF level in synovial fluid than in serum in patients suffering from RA (Cheema et al., Arthritis Rheum., 44, 1313-1319, (2001)), expression of BAFF in salivary gland-infiltrating leukocytes in patients with SS (Groom et al., J. Clin. Invest. 109, 59-68, (2002)), correlation between serum BAFF level in patients suffering from SLE and immunoglobulin or anti-ds DNA antibody (Zhang et al., J. Immunol. 166, 6-10, (2001)) and correlation between BAFF in patients suffering from RA and rheumatoid factor (Cheema et al. , Arthritis Rheum., 44, 1313-1319, (2001)).

From these facts, it can be said that measurement of BAFF in serum or tissue (for example, synovial fluid in patients suffering from RA) not only in patients with autoimmune diseases such as SLE, RA and SS but also in patients before onset of these diseases is useful for prehension of the progress of clinical state, that is, for diagnosis. Autoimmune diseases such as SLE, RA and SS can also be prevented and treated by inhibiting the functions of BAFF.

For measurement of a protein such as BAFF, a method of using an antibody capable of recognizing the protein is general. As antibodies against BAFF, there are actually some commercial products including monoclonal and polyclonal antibodies (for example, antihuman monoclonal antibody (Catalog Number:MAB124) manufactured by R&D Systems, goat antihuman BAFF polyclonal antibody (Catalog Number: SC-5743) manufactured by Santa Cruz Biotech, mouse antihuman BAFF monoclonal antibody (Catalog Number: ALX-804-128-C100) manufactured by ALEXIS) and the like. These antibodies are produced against full-length BAFF or its C-terminal amino acid sequence as antigen, do not exhibit high specificity for human recombinant BAFF in Western blotting, and cannot be satisfactory in respect of detection limit in ELISA, and none of such known antibodies satisfy high-sensitivity diagnosis.

The method of inhibiting functions of BAFF to prevent or treat immune diseases such SLE, RA and SS includes a method of suppressing or inhibiting production of BAFF by suppressing expression of BAFF gene, a method of inhibiting BAFF receptor by a BAFF receptor antagonist or the like, and a method of inhibiting functions of BAFF itself by an anti-BAFF antibody or the like. For example, it is known that the clinical test, by Human Genome Sciences Ltd., of antihuman BAFF (BLyS^{™}) monoclonal antibody (development name: LymophoStat-B^{™}) in SLE or RA patients is advanced to phase-2 clinical test in the US, but the possibility thereof as a therapeutic agent is not necessarily satisfactory and there is demand for development of an antibody having a further useful working effect as a prophylactic or therapeutic agent.
WO 03/055979 discloses an antibody that binds to a protein consisting of amino acid residues 134-285 of BAFF, termed BlyS, as well as competing anti-BAFF antibodies.
WO 03/016469 discloses an antibody binding to a protein which consists of amino acid residues 133-285 of BAFF, termed hTNFSF13b.
US 2002/150,579 discloses an anti-soluble BAFF antibody, termed BlyS. Furthermore, the use of a monoclonal anti-BlyS antibody in a method of treating an autoimmune disease is disclosed as well as a method of monitoring the changes in a condition of patient displaying symptoms of autoimmune disease, specifically systemic lupus erythematosus.
J. Zhang et al., "Cutting edge: a row for B lymphocyte stimulator in systemic lupus erethematosus", Journal of Immunology, Vol. 166, pages 6 to 10 discloses an anti-soluble BAFF antibody, termed BlyS for the treatment and the diagnosis of autoimmune diseases.
Moore et al., "BLyS: member of the tumor necrosis factor family and B lymphocyte stimulator", Science, Vol. 185, No. 5425, pages 262 to 263 discloses identifiers BLyS as member of the human TNF family that induces B cell proliferation and immunoglobulin secretion.

### Disclosure of Invention

The object of the present invention is to provide an antihuman BAFF antibody with higher sensitivity, which can be used in diagnosis of autoimmune diseases. By using such antihuman BAFF antibody, many autoimmune diseases can be more efficiently prevented and treated, and the present invention provides such a pharmaceutical composition and the antibody for use in the prophylactic/therapeutic method. By using such antihuman BAFF antibody, there can also be provided a method of screening a human BAFF inhibiting or activating agent.

Accordingly, the present inventors made extensive study, and as a result, found that when a novel monoclonal antibody (referred to hereinafter as 4H4) prepared by using an antigen having KLH (keyhole limpet hemocyanin) bound to 13 amino acids (SEQ ID NO: 1) as hapten corresponding to a region, in the vicinity of a membrane, of an extracellular domain in the amino acid sequence of human BAFF, is used as a detection antibody in ELISA for detection of human BAFF, detection of surprisingly high sensitivity attaining a detection limit of 0.5 ng/mL is made feasible, and the present invention was thereby completed.
That is, the present invention relates to an antibody, preferably a monoclonal antibody, against a peptide having an amino acid sequence comprising AVQGPEETVT QDC (expressed in single letter amino acid code) in the 134- to 146-positions in human BAFF (B cell activating factor belonging to the TNF family) protein for use in the prevention/treatment of an autoimmune disease as well as to the use of said antibody for the in vitro diagnosis of an autoimmune disease.

That is, the present invention relates to a pharmaceutical composition comprising the antibody of the invention described above and a pharmaceutically acceptable carrier, preferably a pharmaceutical composition for use in preventing and treating autoimmune diseases. Further, the present invention relates to the antibody of the invention for use in preventing or treating autoimmune diseases.

The present invention also relates to the use of the antibody of the invention or a composition comprising said antibody for a diagnostic method.

Further, the present invention relates to a method of detecting or quantifying BAFF in a sample, which includes adding the antibody of the present invention to a sample and measuring BAFF bound to the antibody.

The present invention also relates to a method of screening the inhibiting action or activating action of a test substance on BAFF, which includes adding a test substance in a sample comprising BAFF and measuring, by the antibody of the present invention, a change in the amount of BAFF upon addition of the test substance.

### Brief Description of Drawings

FIG. 1 is a drawing of recombinant human BAFF (manufactured by Chemicon) was detected by conventional Western blotting with the antihuman BAFF monoclonal antibody (4H4) of the invention and a control rabbit antihuman BAFF polyclonal antibody (Chem) manufactured by Chemicon.

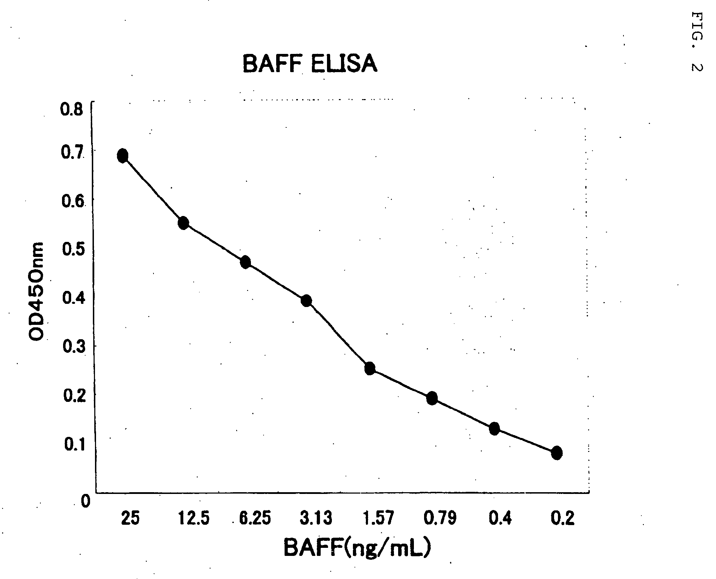

FIG. 2 is a graph showing a standard curve prepared with recombinant human BAFF (manufactured by Chemicon) by ELISA established by using 4H4.
FIG. 3 is a graph showing the effect of 4H4 on IgG production induced by stimulating healthy person- or SLE patient-derived PBL with anti-CD3 antibody.
FIG. 4 is a graph showing the effect of 4H4 on IFNγ production induced by stimulating healthy person- or SLE patient-derived PBL with anti-CD3 antibody.
FIG. 5 is a graph showing the effect of 4H4 on TNF α production induced by stimulating healthy person- or SLE patient-derived PBL with anti-CD3 antibody.

### Best Mode for Carrying out the Invention

The antibody of the present invention is characterized by being produced by using an antigen comprising a peptide comprising the following amino acid sequence (see SEQ ID NO: 1) :
AVQGPEETVT QDC
in the 134- to 146-positions corresponding to a region, in the vicinity of a membrane, of an extracellular domain in the amino acid sequence of human BAFF comprising 285 amino acids.
Usual antibodies are produced by using, as antigen, an N- or C-terminal amino acid sequence of a protein. This is because an amino acid sequence in the vicinity of the center of a protein is sometimes located stereoscopically in the protein interior and is thus sometimes unsuitable as antigen. The Nor C-terminal side of a protein is a stereoscopically exposed structure in many cases, and it is well-known that an antibody against the N- or C-terminal side has sufficient sensitivity in many cases. Accordingly, an antihuman BAFF antibody has been conventionally produced against an amino acid sequence of the N- or C-terminal side. However, the present inventors found that the antihuman BAFF antibody thus produced does not necessarily have sufficient sensitivity. The reasons for this are not fully clarified, but it is expected that the N- or C-terminal side of human BAFF, unlike usual protein, has a structure which is not rendered sufficiently exposed. That is, the reason that the antihuman BAFF antibody produced against an N- or C-terminal side amino acid sequence does not necessarily have sufficient sensitivity is considered attributable to a 3-dimensional structure unique to human BAFF, but is not fully elucidated. It is expected that elucidation of the 3-dimensional structure of human BAFF in the future will clarify the details.

The antibody of the present invention is characterized by using, as antigen, a peptide including an amino acid sequence in an almost central part of human BAFF, and the fact that the amino acid sequence in such a central part is suitable as the antigen must be surprising and is estimated to be attributable to a 3-dimensional structure unique to human BAFF protein.

The antibody of the present invention is characterized by using the above peptide as antigen, and the antibody may be a polyclonal or monoclonal antibody, preferably a monoclonal antibody from the viewpoint of specificity.

The antibody of the present invention comprises a naturally occurring antibody obtained by immunizing a nonhuman mammal with the antigen, according to a usual manner of producing an antibody, a recombinant chimera monoclonal antibody and recombinant human monoclonal antibody (CDR-grafted antibody) which can be produced by using recombinant DNA techniques, and a human antibody which can be produced by a human antibody-producing transgenic animal and the like. The monoclonal antibody comprises monoclonal antibodies having any isotypes of IgG, IgM, IgA, IgD and IgE. IgG or IgM is preferable.

More specifically, the antihuman BAFF monoclonal antibody of the invention is produced according to an existing general production method by immunizing a mammal, preferably a mouse, rat, hamster, guinea pig, rabbit, chicken, cat, dog, pig, goat, horse or bovine, more preferably a mouse, rat, hamster, guinea pig or rabbit, with an antigen having KLH bound to 13 amino acids (SEQ ID NO: 1) as hapten corresponding to a region, in the vicinity of a membrane, of an extracellular domain in the amino acid sequence of human BAFF, if necessary together with Freund's adjuvant, thereby yielding cells (cells from such as the spleen, lymph node, bone marrow or tonsilla, preferably B cells from the spleen) producing the antibody, then preparing hybridomas by using the cells and cells of the bone marrow (myeloma cells) not having an ability to produce an antibody, cloning the hybridomas and selecting them for a clone producing a monoclonal antibody showing specific affinity for the antigen used in immunization of the mammal by immunoassays (ELISA or the like) .

Specifically, the monoclonal antibody of the present invention can be produced in the following manner. That is, a mouse, rat, hamster, guinea pig, chicken or rabbit, preferably a mouse, rat or hamster (including a transgenic animal, created as to produce an antibody derived from other animal, for example, a human antibody-producing transgenic mouse) is subjected to immune-sensitization by injecting or transplanting an antigen having KLH (keyhole limpet hemocyanin) bound to 13 amino acids (SEQ I D NO: 1) as hapten corresponding to a region, in the vicinity of a membrane, of an extracellular domain in the amino acid sequence of human BAFF, if necessary together with Freund's adjuvant, once to several times subcutaneously, intramuscularly, intravenously, intraperitoneally or via a foot pad. Usually, the mammal is immunized once to four times every about 1 to 14 days after first immunization, and from the immune-sensitized animal about 1 to 5 days after final immunization, antibody-producing cells can be obtained and used to yield a clone producing the monoclonal antibody, according to the method described above.

Preferably, the monoclonal antibody of the present invention can be produced as follows. That is, 100 µL of 1 mg/mL aqueous solution of the above antigen peptide in physiological saline, together with Freund's complete adjuvant, is emulsified by sonication and used in intraperitoneally immunizing a mouse (Balb/c. 6-week-old). 100 µL of 1 mg/mL aqueous solution of the antigen peptide in physiological saline and Freund' s complete adjuvant, which were emulsified by sonication, are used as booster for additional immunization 2 weeks after first immunization, followed by additional immunization twice at 2-week intervals, and thereafter, a clone producing the monoclonal antibody can be obtained by the method described above.

Preparation of the hybridoma secreting a monoclonal antibody can be carried out according to the method of Kehler and Milstein (Nature 256, 495-497 (1975)) or a modification thereto. That is, the hybridoma is prepared by subjecting antibody-producing cells contained in the spleen, lymph node, bone marrow or tonsilla, preferably the spleen, obtained from a mammal immune-sensitized as descried above, to cell fusion with a myeloma cell not having an ability to produce an autoantibody, derived from a mammal, preferably a mouse, rat, guinea pig, hamster, rabbit or human, more preferably a mouse, rat or human.

The myeloma cell usable in cell fusion includes, for example, mouse-derived myeloma P3/X63-AG8.653 (653; ATCC No. CRL1580), P3/NSI/1-Ag4-1 (NS-1), P3/X63-Ag8.U1 (P3U1), SP2/O-Ag14 (Sp2/0, Sp2), PAI, FO and BW5147, rat-derivedmyeloma 210RCY3-Ag.2.3, human-derived myeloma U-266AR1, GM1500-6TG-A1-2, UC729-6, CEM-AGR, D1R11 and CEM-T15.

Screening of a hybridoma clone producing the monoclonal antibody can be carried out by culturing the hybridoma in for example a microtiter plate and then measuring, by RIA or enzyme immunoassay such as ELISA, the reactivity of a culture supernatant in a well showing proliferation, with the immune antigen used in the immune sensitization of a mouse described above. Production of the monoclonal antibody from the hybridoma can be carried out in vitro, or in vivo in ascites fluid in a mouse, rat, guinea pig, hamster or rabbit, preferably a mouse or rat, more preferably a mouse, and the monoclonal antibody can be isolated from the resulting culture supernatant or the ascites fluid of the mammal. In the case of in vitro culture, the hybridoma is proliferated, maintained and stored depending on various conditions such as the properties of the cell cultured, the object of test and study, and the culture method, and every nutrient medium such as a known nutrient medium used in producing the monoclonal antibody or a nutrient medium derived from a known basal medium can be used.

The basal medium includes, for example, low-calciummedium such as Ham' s F12 medium, MCDB153 medium or low-calcium MEM medium and high-calciummedium such as MCDB104 medium, MEMmedium, D-MEM medium, RPMI1640 medium, ASF104 medium or RD medium, and the basal medium can contain, for example, serum, hormone, cytokine and/or various inorganic or organic substances if necessary. Isolation and purification of the monoclonal antibody can be carried out by subjecting the culture supernatant or the ascites fluid to precipitation with saturated ammonium sulfate, precipitation with euglobulin, a caproic acid method, a caprylic acid method, ion-exchange chromatography (DEAE or DE52) or affinity column chromatography on an anti-immunoglobulin column or protein A column. From the hybridoma, a gene encoding the monoclonal antibody is cloned and used to produce, by using the transgenic animal producing technique, a transgenic rabbit, goat, sheep or pig in which the antibody encoding gene was integrated in the endogenous gene, and from milk of the transgenic animal, the monoclonal antibody derived from the antibody gene can be obtained in a large amount (Nikkei Science, pp. 78-84, April issue, 1997).

The antihuman BAFF monoclonal antibody of the invention obtained as described above has a surprising feature that 0.5 mg/mL detection limit can be attained as shown in the Examples described later, and thus it is possible to realize not only a highly sensitive method for diagnosing an autoimmune disease but also a prophylactic/therapeutic method for an autoimmune disease and a method of screening a human BAFF inhibiting or activating agent. In the Examples, the antihuman BAFF monoclonal antibody of the invention can be utilized as the antibody, an antibody fragment and a derivative thereof. The antihuman BAFF monoclonal antibody of the present invention can also be used in purification of BAFF derived from cells or blood. The human BAFF protein and a derivative thereof purified by using the antihuman BAFF monoclonal antibody of the present invention can be utilized as a reagent or a pharmaceutical preparation such as B-cell activator. Further, the antihuman BAFF monoclonal antibody of the present invention, a fragment of the antibody and a derivative thereof can be utilized to make an image of BAFF protein by techniques known in the art, such as immunostaining.

The pharmaceutically acceptable carrier in the pharmaceutical composition for use of the present invention includes an excipient, a diluent, an extender, a disintegrating agent, a stabilizer, a preservative, a buffer agent, an emulsifier, an aromatic substance, a coloring agent, a sweetener, a viscous agent, a taste corrective, a solubilizing agent or other additives. By using one or more of such carriers, it is possible to prepare pharmaceutical compositions in the form of tablets, pills, powder, granules, an injection, a solution, capsules, a troche, an elixir, a suspension, an emulsion or a syrup. The pharmaceutical composition of the present invention can be parenterally administered. The form of the parenteral administration includes eye drops and nose drops in addition to an injection formulated in a usual manner, a suppository and pessary for enteric administration, comprising the antibody of the present invention.

The dose of the active ingredient in the pharmaceutical composition for use of the present invention varies depending on the age, sex, weight and condition of the patient, the therapeutic effect, administration method and treatment time; usually, the active ingredient can be administered to an adult in an amount in the range of 1 µg to 1000 mg, preferably 10 µg to 500 mg, for each administration. However, the dose varies depending on various conditions, and thus a dose lower than the above dose may be sufficient in some cases, or a dose higher than the above dose may be necessary in other cases. For example, an injection can be produced by dissolving or suspending the antibody in a nontoxic pharmaceutically acceptable carrier such as physiological saline or distilled water for injection at a concentration of 0. 1 µg antibody/mL carrier to 10 mg antibody/mL carrier.

The injection thus produced can be administered in a dose of 1 µg to 100 mg, preferably 50 µg to 50 mg, for each administration, per body kg once to several times per day to a patient in need of treatment. The administration form can be exemplified by medically suitable administration forms such as intravenous injection, subcutaneous injection, intradermal injection, intramuscular injection and intraperitoneal injection. The administration form is preferably intravenous injection. The injection can also be prepared as a suspension or emulsion with a non-aqueous diluent (for example, propylene glycol, polyethylene glycol and vegetable oils such as olive oil and alcohols such as ethanol) depending on the case. Sterilization of such injections can be carried out by filter sterilization (that is, through a bacteria-retaining filter) or with a sterilizer or through irradiation. That is, a germ-free solid composition is produced by lyophilization and the like can be dissolved in germ-free distilled water for injection or other solvent just before use.

The pharmaceutical composition for use of the present invention is useful for prophylaxis/therapy of autoimmune diseases such as systemic lupus erythematosus (SLE), chronic rheumatoid arthritis (RA), Sjogren's syndrome (SS), autoimmune diabetes,
or an autoimmune disease accompanied by B-cell activation.

The diagnostic agent of the present invention comprises the antibody of the present invention, and contains various reagents binding specifically and highly sensitively to BAFF protein in a sample thereby enabling measurement of a formed complex. The diagnostic composition of the present invention comprises the diagnostic agent of the present invention or the diagnostic agent and a carrier. The composition of the present invention can also be labeled so as to enable measurement of the antibody. For such labeling, a usual method of using a radioactive element or a fluorescent substance can also be used.

In the present invention, the use of the antibody of the invention in diagnosing an autoimmune disease can examine, for example, the progress of an autoimmune disease by establishing an ELISA system with the antihuman BAFF monoclonal antibody of the invention and measuring, in this ELISA system, the concentration of BAFF in serum or tissue collected from a subject such as a patient with an autoimmune disease, as described later in the Examples. The diagnostic use can also be utilized in monitoring for knowing the therapeutic effect or in prediction of prognosis.

In the present invention, the method of detecting or quantifying BAFF can be carried out by adding the antibody of the present invention to a sample and measuring BAFF bound to the antibody. In the method of measuring BAFF bound to the antibody of the present invention, a wide variety of known techniques of detection or quantification by the antigen-antibody reaction can be used, and for example, the ELISA method can be used.
BAFF in the diagnostic use or the detection or quantification method according to the present invention is preferably human BAFF.
The prophylactic/therapeutic antibody for use for autoimmune diseases in the present invention can realize the prophylaxis/therapy of autoimmune diseases by administering the antihuman BAFF monoclonal antibody of the invention together with a pharmaceutically acceptable carrier to patients in need of the prophylaxis/therapy of autoimmune diseases.

The method of screening a human BAFF inhibiting or activating agent in the present invention can be realized by establishing an ELISA system with the antihuman BAFF monoclonal antibody of the invention and then measuring, in this ELISA system, the BAFF binding activity of a test sample with a BAFF receptor-expressing cell or a BAFF receptor protein or its fragment and a derivative thereof, as described later in the Examples. Screening of a human BAFF production inhibiting or promoting agent can be carried out by contacting a test sample with a human BAFF-producing cell and measuring BAFF produced by the cell, by a conventional immunological technique known in the art, such as ELISA.

The antihuman BAFF monoclonal antibody of the present invention is extremely excellent in practical utility because of its surprising feature that the detection limit of 0.5 mg/mL can be attained as shown in the Examples shown later. The fact that the antibody obtained from a peptide comprising an amino acid sequence in the vicinity of in the center of the membrane protein can be an antibody very excellent in specificity and affinity is also beyond expectation because of the unique feature of BAFF.

The antibody of the present invention is characterized by the site used as antigen against it, is extremely excellent in specificity and affinity (sensitivity), and is capable of realizing not only practical method for diagnosing an autoimmune disease but also prophylactic/therapeutic methods for autoimmune diseases, further a method of screening a human BAFF inhibiting or activating agent.
An excellent pharmaceutical composition and diagnostic agent for autoimmune diseases can be provided by using the antibody of the present invention.

### Examples

Hereinafter, the present invention is described in more detail by reference to the Examples, but the present invention is not limited by the Examples.

### Example 1

### Preparation of antihuman BAFF antibody

13 amino acids corresponding to a region, in the vicinity of a membrane, of an extracellular domain in 285 amino acids of BAFF shown in SEQ NO: 2 in the Sequence Listing were selected, then conjugated with KLH by the MBS method, and used as antigen. 100 µL of 1 mg/ml aqueous solution of the antigen peptide in physiological saline and Freund's complete adjuvant were formed into an emulsion by sonication and then used in intraperitoneally immunizing a mouse (Balb/c, 6-week-old). After 2 weeks, 100 µL of 1 mg/ml of an aqueous solution of the antigen peptide in physiological saline and Freund's complete adjuvant, which had been emulsified by sonication, were used as booster for additional immunization, followed by additional immunization twice at 2-week intervals. Two months after the first immunization, the spleen was excised, and lymphocytes were separated in RPMI 1640 medium (containing penicillin and streptomycin). The separated lymphocytes were fused with mouse bone marrow-derivedmyeloma cell P3U1 strain by the polyethylene glycol (PEG) method to prepare hybridoma cells. The hybridoma cells were suspended in a feeder cell-containing HAT medium, pipetted to a 96-well plate (Greiner) and cultured for 15 days. A culture supernatant was recovered from the wells in which the hybridoma cells had been cultured, and antibody-producing cells reactive with the antigen peptide were selected by ELISA (enzyme-linked immunosorbent assay). That is, first, 50 µL of 10 µg/mL antigen peptide was put to each well of the 96-well plate, adsorbed onto the bottom at 4°C overnight and blocked with 100 µL of 2% BSA/PBS at 37°C for 2 hours. Each well was reacted at 4°C overnight with 100 µl supernatant of the hybridoma cells and then reacted at 37°C for 1 hour with a 1000-fold dilution of HRP-labeled anti-mouse IgG and colored with orthophenylene diamine as substrate. After the reaction was terminated with 50 µL of 2 N sulfuric acid, each well was measured for absorption at 492 nm, and hybridomas showing an absorption of 1.0 or more were selected and cloned by limiting dilution.

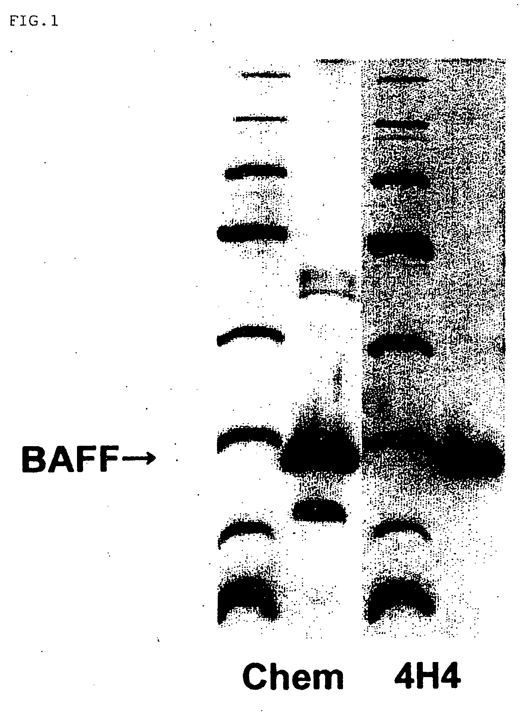

A mouse (Balb/c) to which 0.5 mL pristane had been intraperitoneally administered before 7 days and before 3 days, and the selected hybridoma cells were injected intraperitoneally to the mouse, and about 10 days later, the ascites fluid was collected. The collected ascites fluid was left at room temperature for 30 minutes, then left at 4°C overnight, centrifuged at 15 Krpm for 10 minutes to recovery a supernatant from which a mouse IgG fraction was separated and purified through a protein A-Sepharose column.
By the method described above, a hybridoma cell strain producing the antihuman BAFF antibody (4H4 whose isotype is IgG1), as well as the antibody (4H4), was obtained. The resulting 4H4, and a control rabbit antihuman BAFFpolyclonal antibody (AB16530 : shown as Chem in FIG. 1) manufactured by Chemicon, were used in detection of recombinant human BAFF (manufactured by Chemicon) by the usual Western blotting method. As a result, a 17-KDa band corresponding to the soluble human BAFF was confirmed as shown in FIG. 1.

### Example 2

### Establishment of ELISA

A 96-well plate was coated at 4°C overnight in a volume of 1 µg/well with a rabbit antihuman BAFF polyclonal antibody (AB16530, manufactured by Chemicon) as primary antibody. Each well was washed 3 times with PBS containing 0.05% Tween 20, and then Block Ace (Dainippon Pharmaceutical Co., Ltd.) was added in a volume of 150 µL/well and reacted at 37°C for 2 hours. Each well was washed 3 times with PBS containing 0.05% Tween 20, and 50 µL of sample and 50 µl (8 ng/mL) of biotin-labeled 4H4 were added and reacted at room temperature for 2 hours. Each well was washed 3 times with PBS containing 0.05% Tween 20, and 50 µL of a 1000-fold dilution of streptavidin-labeled HRP (horse radish peroxide) diluted with PBS containing 0.05% Tween 20 was added and reacted at room temperature for 30 minutes. Each well was washed 5 times with PBS containing 0.05% Tween 20, and then 50 µL of TMB One Solution (manufactured by Clonetech) was added and reacted for 5 minutes at room temperature, then 50 µL of 1 N HCl was added, and the each well was measured for absorbance at 450 nm with a plate reader (manufactured by Perkin Elmer). As a standard substance, recombinant human BAFF (manufactured by Chemicon) was used to prepare a standard curve.

As a result, establishment of ELISA system showing an excellent linear relationship at a BAFF concentration of from 25 ng/mL to 0.2 ng/mL was confirmed. Particularly, while a concentration in the range of 2 ng/mL to 0.5 ng/mL cannot be detected by conventional antihuman BAFF antibody, such reliable detection at this concentration reveals that the diagnosis of autoimmune diseases by using the antihuman BAFF monoclonal antibody of the present invention or the screening of a BAFF inhibiting or activating agent is very useful.

### Example 3

### Action on human PBL '

Blood was collected from a healthy person and patients diagnosed as having SLE, and a lymphocyte layer was separated and collected therefrom by gravity centrifugation with Ficoll, to give Peripheral Blood Lymphocytes (PBLs). PBLs were suspended in RPMI1640 medium containing 10% FBS (Fetal bovine serum), and anti-CD3 antibody diluted at 10 µg/mL with PBS was put to a 24-well culture plate and adsorbed at 4°C overnight onto the bottom, and PBLs were inoculated at 5×10⁵ cells/well. Simultaneously, 4H4 was added at a final concentration of 10 µg/mL, followed by culture for 4 days or 7 days at 37°C in 7% CO₂ in a CO₂ incubator. The culture supernatant was collected and measured by sandwich ELISA method using monoclonal antibodies (IgG, primary antibody, manufactured by BDPharmingen, Cat. No. 555784; secondary antibody (biotin-labeled), manufactured by BDPharmingen, Cat. No. 555785; IFNγ, primary antibody, manufactured by BDPharmingen, Cat. No. 554698; secondary antibody (biotin-labeled), manufactured by BDPharmingen, Cat. No. 554550; TNFα, primary antibody, manufactured by BDPharmingen, Cat. No. 551220; and secondary antibody (biotin-labeled), manufactured by BDPharmingen, Cat. No. 554511) reacting specifically with IgG, IFNγ and TNFα in the culture supernatant, respectively.

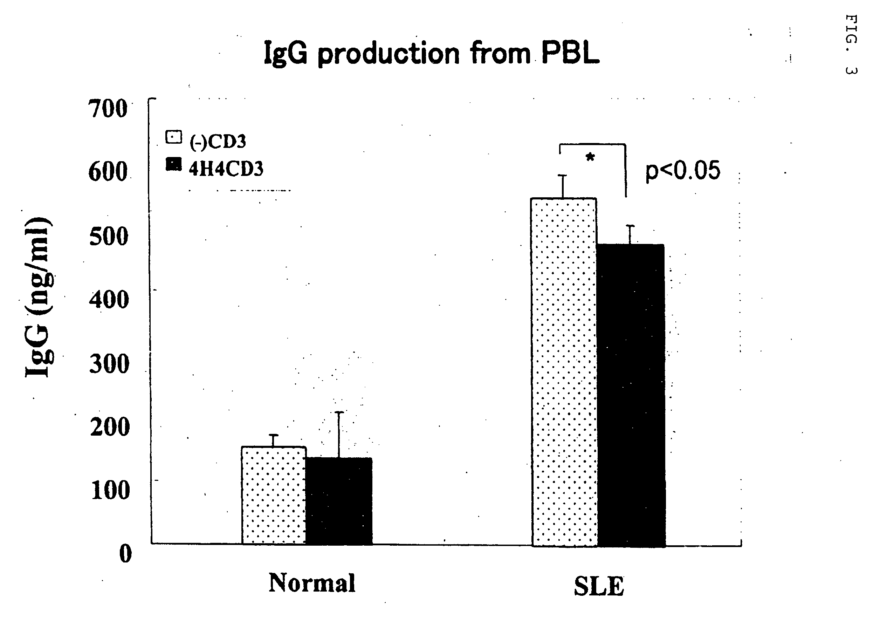

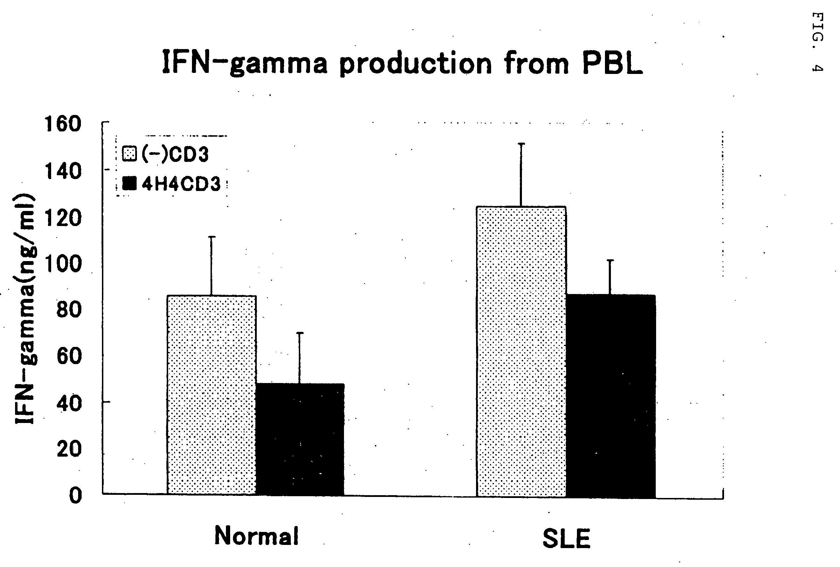

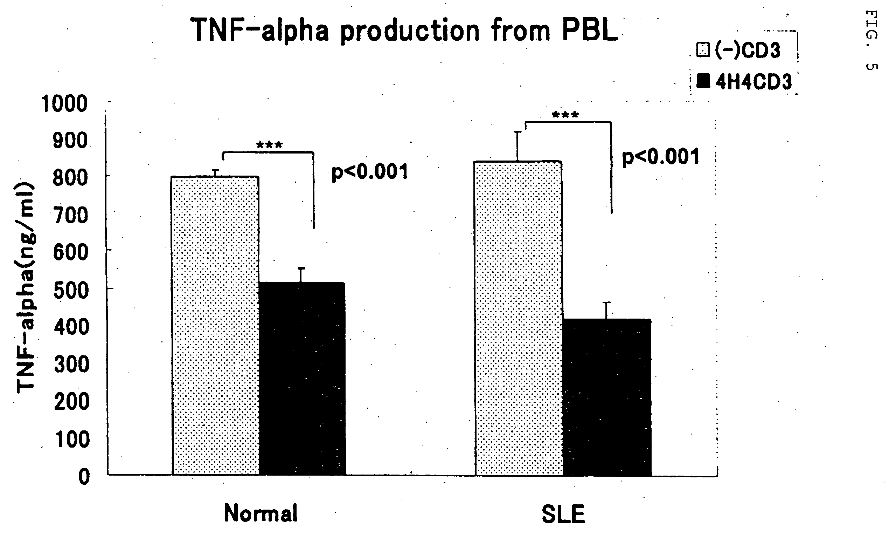

The results are shown in FIGS. 3 to 5. Production of IgG (FIG. 3), IFNγ (FIG. 4) and TNFα (FIG. 5) induced by stimulation with anti-CD3 antibody (all of which are shown in the left (open) column in the graph) from PBL derived from a healthy person and an SLE patient was regulated by addition of 4H4 (all of which are shown in the right (solid) column in the graph) . Particularly, it was revealed that the induction of production of IgG, IFN-γ and TNF-α by anti-CD3 antibody is higher in patients than in healthy person, and the degree of suppression of production thereof by 4H4 is also high. Particularly, it was revealed that the production of TNFα by this antibody is significantly suppressed (t-test). Promotion of such production is considered to participate considerably in formation of clinical state, and suppression of such production is estimated to be a new attempt at therapy of autoimmune diseases such as SLE. Accordingly, it was suggested that the antihuman BAFFmonoclonal antibody of the present invention is very useful for development of therapeutic agents for autoimmune diseases.

### Industrial Applicability

The present invention is industrially applicable because it provides an extremely specific and highly sensitive antibody to BAFF, particularly human BAFF, which comes to be revealed to have relationship with autoimmune diseases such as systemic lupus erythematosus (SLE), chronic rheumatoid arthritis (RA), Sjogren's syndrome (SS), autoimmune diabetes or an autoimmune disease accompanied by B-cell activation, and cannot only be used for therapy, prophylaxis and diagnosis of autoimmune diseases, but also provides a method of screening a new substance useful for therapy and prophylaxis of autoimmune diseases.

## Claims

1. An antibody directed against the peptide consisting of an amino acid sequence AVQGPEETVT QDC (expressed in single letter amino acid code) which is the amino acid sequence of the 134- to 146-positions in human BAFF (B cell activating factor belonging to the tumor necrosis factor family) protein or a pharmaceutical composition comprising said antibody together with a pharmaceutically acceptable carrier, for use in the prevention or treatment of an autoimmune disease.

2. The antibody or pharmaceutical composition for use according to claim 1, wherein the antibody is a monoclonal antibody.

3. The antibody or pharmaceutical composition for use according to one of claims 1 or 2, wherein the autoimmune disease is systemic lupus erythematosus, chronic rheumatoid arthritis, Sjogren's syndrome, autoimmune diabetes, or an autoimmune disease accompanied by B-cell activation.

4. Use of an antibody directed against the peptide consisting of an amino acid sequence AVQGPEETVT QDC (expressed in single letter amino acid code) which is the amino acid sequence of the 134-to-146-positions in human BAFF (B cell activating factor belonging to the tumor necrosis factor family) protein or a pharmaceutical composition comprising said antibody together with a pharmaceutically acceptable carrier for the in vitro diagnosis of an autoimmune disease.

5. The antibody or pharmaceutical composition for use according to claim 4, wherein the antibody is a monoclonal antibody.

6. The antibody or pharmaceutical composition for use according to one of claims 4 or 5, wherein the autoimmune disease is systemic lupus erythematosus, chronic rheumatoid arthritis, Sjogren's syndrome, autoimmune diabetes, or an autoimmune disease accompanied by B-cell activation.

7. The antibody or pharmaceutical composition for use according to one of claims 4 or 5, wherein the diagnosis is carried out by ELISA.

8. A method of detecting or quantifying BAFF in a sample, which comprises adding the antibody used in any of claim 1 or 2 to the sample and measuring the amount of BAFF bound to the antibody.

9. The method according to claim 8,
wherein BAFF is human BAFF.

10. The method according to claim 8 or 9,
wherein the method of measuring the amount of BAFF bound to the antibody is carried out by ELISA.

11. A method of screening a test substance having the inhibiting or activating action on BAFF, which comprises adding the test substance to a sample containing BAFF and measuring a change, upon addition of the test substance, in the amount of BAFF by the antibody used in claim 1 or 2.

12. The method according to claim 11, wherein BAFF is human BAFF.

13. The method according to claim 11 or 12,
wherein the method of screening the inhibiting action on BAFF is screening of a prophylactic/therapeutic agent for autoimmune diseases.

## Patentansprüche

1. Antikörper, der gegen das Peptid, bestehend aus einer Aminosäuresequenz AVQGPEETVT QDC (ausgedrückt im EinBuchstaben-Aminosäure-Code), die die Aminosäuresequenz der 134-bis-146-Positionen in humanem BAFF (B-ZellAktivierungsfaktor, der zu der Tumornekrosefaktor-Familie gehört)-Protein ist, gerichtet ist, oder pharmazeutische Zusammensetzung, die den Antikörper zusammen mit einem pharmazeutisch annehmbaren Träger umfasst, zur Verwendung bei der Prävention oder Behandlung einer Autoimmunerkrankung.

2. Antikörper oder pharmazeutische Zusammensetzung zur Verwendung gemäß Anspruch 1, wobei der Antikörper ein monoklonaler Antikörper ist.

3. Antikörper oder pharmazeutische Zusammensetzung zur Verwendung gemäß einem der Ansprüche 1 oder 2, wobei die Autoimmunerkrankung systemischer Lupus erythematodes, chronische rheumatoide Arthritis, Sjögrens Syndrom, Autoimmun-Diabetes oder eine Autoimmunerkrankung, verbunden mit B-ZellAktivierung, ist.

4. Verwendung eines Antikörpers, der gegen das Peptid, bestehend aus einer Aminosäuresequenz AVQGPEETVT QDC (ausgedrückt im Ein-Buchstaben-Aminosäure-Code), die die Aminosäuresequenz der 134-bis-146-Positionen in humanem BAFF (B-Zell-Aktivierungsfaktor, der zur Tumornekrosefaktor-Familie gehört)-Protein ist, gerichtet ist, oder einer pharmazeutischen Zusammensetzung, die den Antikörper zusammen mit einem pharmazeutisch annehmbaren Träger umfasst, für die In-vitro-Diagnose einer Autoimmunerkrankung.

5. Antikörper oder pharmazeutische Zusammensetzung zur Verwendung gemäß Anspruch 4, wobei der Antikörper ein monoklonaler Antikörper ist.

6. Antikörper oder pharmazeutische Zusammensetzung zur Verwendung gemäß einem der Ansprüche 4 oder 5, wobei die Autoimmunerkrankung systemischer Lupus erythematodes, chronische rheumatoide Arthritis, Sjögrens Syndrom, Autoimmun-Diabetes oder eine Autoimmunerkrankung, verbunden mit B-ZellAktivierung, ist.

7. Antikörper oder pharmazeutische Zusammensetzung zur Verwendung gemäß einem der Ansprüche 4 oder 5, wobei die Diagnose durch ELISA durchgeführt wird.

8. Verfahren zum Detektieren oder Quantifizieren von BAFF in einer Probe, das Zusetzen des Antikörpers, der in Anspruch 1 oder 2 verwendet wird, zu der Probe und Messen der Menge an BAFF, die an den Antikörper gebunden ist, umfasst.

9. Verfahren gemäß Anspruch 8, wobei BAFF humaner BAFF ist.

10. Verfahren gemäß Anspruch 8 oder 9, wobei das Verfahren zum Messen der Menge an BAFF, die an den Antikörper gebunden ist, durch ELISA durchgeführt wird.

11. Verfahren zum Screening einer Testsubstanz, die inhibierende oder aktivierende Wirkung auf BAFF hat, das Zusetzen der Testsubstanz zu einer Probe, die BAFF enthält, und Messen einer Änderung, nach Zugabe der Testsubstanz, bei der Menge an BAFF durch den Antikörper, der in Anspruch 1 oder 2 verwendet wird, umfasst.

12. Verfahren gemäß Anspruch 11, wobei BAFF humaner BAFF ist.

13. Verfahren gemäß Anspruch 11 oder 12, wobei das Verfahren des Screenings der inhibierenden Wirkung auf BAFF Screening eines prophylaktischen/therapeutischen Mittels für Autoimmunerkrankungen ist.

## Revendications

1. Anticorps dirigé contre le peptide constitué d'une séquence d'acides aminés AVQGPEETVT QDC (exprimée selon le code à une seule lettre des acides aminés) qui est la séquence d'acides aminés correspondant aux positions 134 à 146 dans la protéine BAFF humaine (facteur d'activation des lymphocytes B appartenant à la famille des facteurs de nécrose tumorale) ou composition pharmaceutique contenant ledit anticorps conjointement avec un support pharmaceutiquement acceptable, pour une utilisation dans la prévention ou le traitement d'une maladie auto-immune.

2. Anticorps ou composition pharmaceutique pour une utilisation selon la revendication 1, dans lequel/laquelle l'anticorps est un anticorps monoclonal.

3. Anticorps ou composition pharmaceutique pour une utilisation selon la revendication 1 ou la revendication 2, dans lequel/laquelle la maladie autoimmune est le lupus érythémateux disséminé, la polyarthrite rhumatoïde chronique, le syndrome de Sjögren, le diabète auto-immun ou une maladie autoimmune accompagnée d'une activation des lymphocytes B.

4. Utilisation d'un anticorps dirigé contre le peptide constitué d'une séquence d'acides aminés AVQGPEETVT QDC (exprimée selon le code à une seule lettre des acides aminés) qui est la séquence d'acides aminés correspondant aux positions 134 à 146 dans la protéine BAFF (facteur d'activation des lymphocytes B appartenant à la famille des facteurs de nécrose tumorale) humain ou d'une composition pharmaceutique contenant ledit anticorps conjointement avec un support pharmaceutiquement acceptable pour le diagnostic in vitro d'une maladie auto-immune.

5. Anticorps ou composition pharmaceutique pour une utilisation selon la revendication 4, dans lequel/laquelle l'anticorps est un anticorps monoclonal.

6. Anticorps ou composition pharmaceutique pour une utilisation selon la revendication 4 ou la revendication 5, dans lequel/laquelle la maladie autoimmune est le lupus érythémateux disséminé, la polyarthrite rhumatoïde chronique, le syndrome de Sjögren, le diabète auto-immun ou une maladie autoimmune accompagnée d'une activation des lymphocytes B.

7. Anticorps ou composition pharmaceutique pour une utilisation selon la revendication 4 ou la revendication 5, dans lequel/laquelle le diagnostic est réalisé par ELISA.

8. Procédé de détection ou de quantification d'un BAFF dans un échantillon, comprenant l'ajout de l'anticorps utilisé dans l'une quelconque des revendications 1 et 2 à l'échantillon et la mesure de la quantité de BAFF lié à l'anticorps.

9. Procédé selon la revendication 8,
dans lequel le BAFF est le BAFF humain.

10. Procédé selon la revendication 8 ou 9,
dans lequel le procédé de mesure de la quantité de BAFF lié à l'anticorps est réalisé par ELISA.

11. Procédé de criblage d'une substance à tester possédant l'action inhibitrice ou activatrice sur BAFF, comprenant l'ajout de la substance à tester à un échantillon contenant le BAFF et la mesure d'un changement, lors de l'ajout de la substance à tester, de quantité de BAFF par l'anticorps utilisé dans la revendication 1 ou 2.

12. Procédé selon la revendication 11, dans lequel le BAFF est le BAFF humain.

13. Procédé selon la revendication 11 ou 12,
dans lequel le procédé de criblage de l'action inhibitrice sur le BAFF est un criblage d'un agent prophylactique/thérapeutique pour des maladies autoimmunes.


### SEQUENCE LISTING

<110> Kowa Company,Ltd. Takeuchi, Tsutomu Yoshimoto, Keiko
<120> Antihuman BAFF antibody
<130> 17340EP
<140> 05 776 879.8
   <141> 2005-08-30
<150> US 60/605,516
   <151> 2004-8-31
<160> 2
<170> PatentIn version 3.2
<210> 1
   <211> 13
   <212> PRT
   <213> Homo sapiens
<400> 1
   Ala Val Gln Gly Pro Glu Glu Thr Val Thr Gln Asp Cys
   1               5                   10
<210> 2
   <211> 285
   <212> PRT
   <213> Homo sapiens
<400> 2
   Met Asp Asp Ser Thr Glu Arg Glu Gln Ser Arg Leu Thr Ser Cys Leu
   1               5                   10                  15
   Lys Lys Arg Glu Glu Met Lys Leu Lys Glu Cys Val Ser Ile Leu Pro
               20                  25                  30
   Arg Lys Glu Ser Pro Ser Val Arg Ser Ser Lys Asp Gly Lys Leu Leu
           35                  40                  45
   Ala Ala Thr Leu Leu Leu Ala Leu Leu Ser Cys Cys Leu Thr Val Val
       50                  55                  60
   Ser Phe Tyr Gln Val Ala Ala Leu Gln Gly Asp Leu Ala Ser Leu Arg
   65                  70                  75                  80
   Ala Glu Leu Gln Gly His His Ala Glu Lys Leu Pro Ala Gly Ala Gly
                   85                  90                  95
   Ala Pro Lys Ala Gly Leu Glu Glu Ala Pro Ala Val Thr Ala Gly Leu
               100                 105                 110
   Lys Ile Phe Glu Pro Pro Ala Pro Gly Glu Gly Asn Ser Ser Gln Asn
           115                 120                 125
   Ser Arg Asn Lys Arg Ala Val Gln Gly Pro Glu Glu Thr Val Thr Gln
       130                 135                 140
   Asp Cys Leu Gln Leu Ile Ala Asp Ser Glu Thr Pro Thr Ile Gln Lys
   145                 150                 155                 160
   Gly Ser Tyr Thr Phe Val Pro Trp Leu Leu Ser Phe Lys Arg Gly Ser
                   165                 170                 175
   Ala Leu Glu Glu Lys Glu Asn Lys Ile Leu Val Lys Glu Thr Gly Tyr
               180                 185                 190
   Phe Phe Ile Tyr Gly Gln Val Leu Tyr Thr Asp Lys Thr Tyr Ala Met
           195                 200                 205
   Gly His Leu Ile Gln Arg Lys Lys Val His Val Phe Gly Asp Glu Leu
       210                 215                 220
   Ser Leu Val Thr Leu Phe Arg Cys Ile Gln Asn Met Pro Glu Thr Leu
   225                 230                 235                 240
   Pro Asn Asn Ser Cys Tyr Ser Ala Gly Ile Ala Lys Leu Glu Glu Gly
                   245                 250                 255
   Asp Glu Leu Gln Leu Ala Ile Pro Arg Glu Asn Ala Gln Ile Ser Leu
               260                 265                 270
   Asp Gly Asp Val Thr Phe Phe Gly Ala Leu Lys Leu Leu
           275                 280                 285